# EUROPEAN PATENT APPLICATION

(11) **EP 4 578 375 A1**
(43) Date of publication of application: **02.07.2025**
(21) Application number: 22956621.1
(22) Date of filing: 26.12.2022
(51) Int. Cl.: A61B 5/00, A61B 5/12, G16H 50/30, G16H 50/20, G16H 50/70, G10L 15/26, G10L 21/0208

(54) **COGNITIVE ABILITY ASSESSMENT DEVICE, MOBILE TERMINAL, AND VOICE ACQUISITION DEVICE**

(30) Priority: 23.08.2022 KR 20220105435
(71) Applicant: Korea Electrotechnology Research Institute, Changwon-si, Gyeongsangnam-do 51543 (KR)
(72) Inventor: PARK, Young Jin, Changwon-si Gyeongsangnam-do 51543 (KR); JEONG, Kye Young, Changwon-si Gyeongsangnam-do 51543 (KR); CHO, Kyung Hwan, Changwon-si Gyeongsangnam-do 51543 (KR)
(74) Representative: Bryn Aarflot AS
(86) International application number: PCT/KR2022/021263
(87) International publication number: WO 2024/043416

(57) **Abstract**

Examples of the present disclosure can provide evaluation results regarding a user's cognitive ability in a simple manner through an analysis of the linguistic characteristics of the user's vocal data. Mild cognitive impairment or dementia of the user in daily life is easily detected through a convenient test so as to facilitate medical management from the early stages, whereby the quality of life for patients with degenerative brain diseases can be improved.

## Description

### [Technical Field]

Embodiments of the disclosure relate to a cognitive ability assessment device, a mobile terminal, and an acquisition device of utterance and electro encephalo graphy (EEG).

### [Background Art]

Due to advances in medical technology, the average life expectancy of people is increasing. The increasing life expectancy leads to a worldwide increase in the proportion of elderly people in the total population. The elderly population is susceptible to various geriatric diseases, and health care for the elderly population is emerging as a national and social issue.

In particular, with the continuous increase in the elderly population, the number of elderly people suffering from degenerative brain diseases, such as Alzheimer's disease (AD) and Parkinson's disease (PKS), which the elderly fear the most, is continuously increasing. These degenerative brain diseases have no cure once they occur, and they are not only expensive to treat and care, but also drastically worsen the quality of life. Therefore, medical care for the elderly population with these degenerative brain diseases poses a huge personal and social burden.

### [Detailed Description of the Invention]

### [Technical Problem]

Embodiments of the disclosure may provide a method for identifying a decline in cognitive ability through linguistic or language and acoustic or speech characteristics detected through an utterance or EEG in a person's daily life, thereby easily identifying early mild cognitive impairment (MCI) and early dementia. Embodiments of the disclosure may provide a device being capable of collecting an utterance or EEG of elderly people easily in their daily life.

### [Technical Solution]

Embodiments of the disclosure may provide a cognitive ability assessment device, comprising a task provider providing a user with a plurality of tasks during a first test period and a second test period after the first test period, an utterance data collector obtaining utterance data in a digital form, converted from a test speech in which a magnitude of at least a portion of a basic speech according to a user's answer to at least one of the plurality of tasks is adjusted, and a cognitive ability measurer producing an assessment result regarding the user's cognitive ability based on at least one linguistic characteristic of the utterance data.

Embodiments of the disclosure may provide a mobile terminal, comprising a task providing module providing a user with a plurality of tasks during a test period, and EEG signals' acquistion around ear and an utterance data collection module obtaining utterance data in a digital form, converted based on a test speech in which a magnitude of at least a portion of a basic speech according to a user's answer to at least one of the plurality of tasks is adjusted.

The mobile terminal may further comprise a cognitive ability measurement module producing an assessment result regarding the user's cognitive ability based on at least one linguistic characteristic of the utterance data.

Embodiments of the disclosure may provide an utterance acquisition device, comprising an output module outputting a speech according to a plurality of tasks provided to a user, an input module obtaining a basic speech according to the user's answer to at least one of the plurality of tasks, and a processing module analyzing a magnitude or frequency band of the basic speech, adjusting to amplify a magnitude of a basic speech having a small magnitude and not to amplify a large basic speech for which speech recognition is easy through the analysis to generate a test speech to enable speech recognition even for the user's small speech, and converting the test speech into utterance data in a digital form.

The utterance acquisition device may further comprise a communication module transmitting utterance data to an outside.

### [Advantageous Effects]

According to embodiments of the disclosure, it is possible to easily identify changes in cognitive ability from utterances made by the user in everyday life environments rather than in medical facilities and to provide the results of an absolute assessment of the utterer's personal cognitive ability based on the user's personal utterance information, a person's temporal cognitive ability comparison assessment based on periodic assessment information, or an assessment on disease such as cognitive impairment or dementia through a relative comparison of cognitive ability according to a person's age or education level.

### [Brief Description of the Drawings]

FIG. 1 is a view schematically illustrating an example of a configuration of a cognitive ability assessment system according to embodiments of the disclosure;
FIGS. 2 to 5 are views illustrating examples of a method of performing a cognitive ability assessment by a cognitive ability assessment system according to embodiments of the disclosure;
FIG. 6 is a view schematically illustrating another example of a configuration of a cognitive ability assessment system according to embodiments of the disclosure;
FIGS. 7 and 8 are views exemplarily illustrating a configuration of the utterance acquisition device illustrated in FIG. 6;
FIG. 9 is a view schematically illustrating still another example of a configuration of a cognitive ability assessment system according to embodiments of the disclosure;
FIGS. 10 and 11 are views illustrating examples of a method of performing a cognitive ability assessment by a cognitive ability assessment system according to embodiments of the disclosure; and
FIGS. 12 and 13 are flowcharts illustrating examples of a process of a cognitive ability assessment method according to embodiments of the disclosure.

### [Mode for Carrying out the Invention]

Hereinafter, embodiments of the disclosure are described in detail with reference to the accompanying drawings. In assigning reference numerals to components of each drawing, the same components may be assigned the same numerals even when they are shown on different drawings. When determined to make the subject matter of the disclosure unclear, the detailed of the known art or functions may be skipped. As used herein, when a component "includes," "has," or "is composed of" another component, the component may add other components unless the component "only" includes, has, or is composed of" the other component. As used herein, the singular forms "a", "an" and "the" are intended to include the plural forms as well, unless the context clearly indicates otherwise.

Such denotations as "first," "second," "A," "B," "(a)," and "(b)," may be used in describing the components of the present invention. These denotations are provided merely to distinguish a component from another, and the essence, order, or number of the components are not limited by the denotations.

In describing the positional relationship between components, when two or more components are described as "connected", "coupled" or "linked", the two or more components may be directly "connected", "coupled" or "linked" ", or another component may intervene. Here, the other component may be included in one or more of the two or more components that are "connected", "coupled" or "linked" to each other.

When such terms as, e.g., "after", "next to", "after", and "before", are used to describe the temporal flow relationship related to components, operation methods, and fabricating methods, it may include a non-continuous relationship unless the term "immediately" or "directly" is used.

When a component is designated with a value or its corresponding information (e.g., level), the value or the corresponding information may be interpreted as including a tolerance that may arise due to various factors (e.g., process factors, internal or external impacts, or noise).

Hereinafter, various embodiments of the disclosure are described in detail with reference to the accompanying drawings.

Recently, various methods for early detection of dementia have been studied. According to the 4th comprehensive dementia management plan, dementia screening tests, diagnostic tests, and differential tests are conducted. Among these, dementia screening tests are conducted at dementia care centers, and test tools such as MMSE-KC, MMSE-DS, and CIST are used for the test. In the second stage diagnostic test, CERAD-K and SNSB are used for the diagnostic test. If there is an abnormality in these screening and diagnostic tests, PET imaging analysis is performed to conduct a precise test, such as beta protein analysis, to make an accurate disease diagnosis. These tests are precise tests and can be diagnosed directly at the hospital.

The cognitive ability assessment method by the cognitive ability assessment system according to embodiments of the disclosure may be performed in a short time at a welfare center or a place of residence frequently visited by the elderly, not at a designated medical institution. Further, the proposed method may perform a screening high-risk groups for mild cognitive impairment, and by conducting a thorough test and diagnostic test only for the selected high-risk groups, unnecessary test costs for subjects other than high-risk groups may be reduced, and national costs may be reduced because the government only needs to provide governmental support to high-risk groups. Further, the proposed testing method may be easily and conveniently repeated and continuously performed on a regular basis (e.g., every 6 months) for older people anytime, anywhere, so that high-risk groups with early dementia symptoms may be continuously managed and monitored over time rather than conventional one-off methods in medical institutions.

As is known to date, it is difficult to treat or relieve symptoms after progressing to moderate dementia. Therefore, a method of detecting early dementia and slowing the progression of dementia through drug treatment and hospital treatment is being promoted as a good way to enable activities in daily life.

In fact, as an efficient method for detecting early dementia symptoms, a high-risk group for dementia is detected through a screening test, and subjects of the high-risk group are periodically tested and diagnosed, which is more efficient than conventional diagnosis through a test on random ones (about 1 in 10 elderly people) or all of the elderly people. In particular, it would be very useful if a high-risk group with early dementia may be detected early through a continuous screening test, and the high-risk group of elderly people may be actively managed by the government to slow entry into the dementia stage that requires national management.

Recently, several methods have been proposed as methods for selecting such high-risk groups. For example, a method of monitoring the gait of the elderly in their daily lives and a method of monitoring sleep patterns for a long time have been suggested.

As another way, research on monitoring degenerative brain diseases such as dementia, based on utterance in the elderly, is actively underway. There are various pieces of information about a person's health condition in the data (acoustic data or speech data) expressed by speech, such as conversation. For example, a method through analysis of acoustic features (tone, speed, size, etc.) may be proposed. Alternatively, there is also a method of finding acoustic features associated with a disease by converting utterance information into a frequency domain and converging artificial intelligence technology or the like in the frequency domain. Here, an utterance is an answer thereto expressed in a speech when various pieces of information to be heard, asked, or expressed in words are obtained through five senses.

This method may be a method of leading to an utterance by telling or showing prepared questions, or transferring undetermined questions (e.g., daily life questions and conversations) to a subject using a speech or an image, and analyzing utterance data in real time or later through storage, speech recording, and video recording, called tasks. The feature analysis of speech may be mainly to analyze tone, speed, and magnitude. The method using these acoustic features may have excellent screening ability for dementia. Further, it may be necessary to extract common phonetic features considering differences according to dialect use and differences in individual vocal structures.

As another method using conversational or utterance data of older people, there is a method of analyzing linguistic features in answers to questions, such as an answer time to a question, used interjections, used words, a sentence configuration , understanding of language etc. using utterance or conversation data such as a picture description, a daily life description, etc.

In this method, the tester tells or shows the elderly pre-verified questions (called tasks) in a 1:1 face-to-face manner, records the elderly's answers to each question, or stores them in a specific storage space (e.g., a memory of a smart device). To analyze this result, the recorded speech is expressed in text (called transcription), and each answer to each question used is stored to match. At the time of transcription, the time of the elderly's answer to the question for each answer, the number of words used, the accuracy of the sentence composition (e.g., assessment of whether it is in a normal order of the subject, object, and predicate), the appropriateness of the words used, the difficulty of the words used, and the interjections used in the middle are also displayed and transcribed. Until now, the recorded speech is manually converted into text through test, and the data converted into text is organized according to an analysis rule. In addition to the foregoing, this rule may include the degree of repetition of similar words, the limitation of the used words, and, when a word necessary for explanation is not remembered, or an interjection used because the word is not remembered.

Further, for analysis, it is necessary to synchronize the recorded speech section with the transcribed text. This is called labeling. There may also be a tool that labels Hangul.

These methods take many hours or more to collect utterance data, and require that the utterer and the counselor perform a face-to-face test in a specific place such as a hospital, but also take a lot of manpower and time to transfer the collected speech data.

Recently, there has been a lot of research on recognizing uttered speech and analyzing its linguistic and acoustic structure by fusing artificial intelligence (AI) technologies. For example, a speech recognizer may be used to convert the uttered speech data into text. Further, an AI speech recognizer may be used for labeling or transcription.

By using such a speech recognizer, various speech recognition devices and equipment have been developed, and services using the same have been activated. As a device for acquiring speech or utterance information, a smart device capable of exchanging information in two ways such as a smart phone and a tablet, an interactive robot, an artificial intelligence speaker, or the like may be used. The basic configuration of these devices may include a microphone for listening to the speech for the conversation, an artificial intelligence-based speech recognizer for recognizing the input speech, an answer technology based on the recognized speech, and a speaker for finally explaining the question or telling the question by speech.

The current method of collecting utterance information using a microphone built into a smart device such as a portable tablet requires subjects to see or listen to the smart device. As another example, a question for utterance may be provided and utterance information may be obtained even through a device embedded with a fixed artificial intelligence speech recognition function, such as an artificial intelligence speaker, a social robot, or a chatbot. As such, whether it is a portable device or a stationary device, the microphone in the devicefor collecting utterance data and the utterer are away from each other at a predetermined distance. For this reason, the microphone may receive not only the utterer's speech, but also speeches other than the utterer's speech and ambient environmental noise. In this case, the speech recognizers of the artificial intelligence speaker and the social robot may not properly recognize the utterer's speech nor does it convert into text due to poor speech recognition performance in a noise or noise environment. Alternatively, accurate speech recognition or speech data is not converted into text. For example, it has a good recognition rate for the speech uttered from close to the microphone built in the device used for utterance collection has a good speech recognition rate but shows a poor speech recognition rate if away even a little. This is why the magnitude of the input speech decreases as the distance between the utterer and the microphone increases, and other ambient sounds (environmental noise, life noise, and speeches from others than the utterer) are included. In other words, since the spectrum of the input speech includes noise components in addition to the original utterer's speech, there will be many errors in utterance data analysis. This is because noise and the utterer's speech are mixed with each other, and each may not be physically separated.

As a method of solving this problem, there may be a method of collecting only the utterer's speech using a plurality of directional microphones in a smart device. Further, a noise removal algorithm may be embedded in the smart device to remove unnecessary noise or the like from the speech input through the microphone.

Further, if the distance becomes a little farther, it may be inappropriate for the subjects to listen to the questions or conversation. In a case where there is no general assist device or an environment in which external environmental noise is mixed, a problem occurs that the subject may not properly hear the question, and because of this, the subject (utterer) may not answer the question because he/she may not hear it, but there is an error that may be determined as a cognitive impairment in the analysis.

Further, in order for the subject (utterer) to listen to the question and lead the subject to utter, it may be very important for the subject to look at or listen to the question. Embodiments of the disclosure may provide a function of amplifying the question speech to be appropriate for the user so that older people with normal hearing ability as well as hearing impairment may properly hear the question in order for the subject (e.g., an old person) to hear well the question.

In a quiet and non-external noise-free space, normal assessment results may be obtained in the conventional proposed method, but there may be various difficulties in applying related technologies in a space where the elderly are actually active. As another method, a wireless earphone having a microphone function may be used. The questionnaire (tasks) and instructions may be clearly heard through the wireless earphone, or the speech received through the wireless earphone may be transferred to a smart device (smartphone, tablet, computer, etc.). Further, the magnitude of the question speech may be fitted constantly regardless of the status of the speaker in each device, so that the reliability of the test may increase.

However, when this method is also performed in everyday life, the utterer's speech is transferred along with external noise in addition to the utterer's utterance data.

Embodiments of the disclosure may provide a method for overcoming the foregoing problems in the practical use of a screening test technology for cognitive impairment of older people by applying a linguistic/acoustic analysis technology based on utterance data of older people in their daily lives.

Therefore, embodiments of the disclosure may provide a method of easily and conveniently recording utterance data while listening to and viewing questions for test in actual individual living spaces, etc., as well as non-medical public institutions such as an elderly welfare center, etc., which are easily accessible to older people, in order to use language test technology to put to practical use a convenient and efficient cognitive impairment and early dementia screening test technology. Further, it is possible to test and assess even if there is life noise and noise in a daily living space.

Most older people have hearing loss due to aging, so a way to obtain information for speech should also be proposed. It is preferable to perform sound amplification according to the degree of hearing loss in the inspection test. As an example of this method, when an earphone is used, the volume may be adjusted, and when a device such as an earphone is not helpful, it is necessary to appropriately amplify the speech. Accordingly, embodiments of the disclosure may include adjusting the magnitude of a speech suitable for the elderly to listen to a question.

A need also exists for a method of transmitting utterance information to a cloud server as soon as possible so that the utterance data analysis result may be provided to the service proviced immediately after the test, and a method for analyzing the utterance information.

In particular, in order to conduct the test as efficiently and quickly as possible, a technology for selecting an optimal question set for various tasks is also required. Further, a technology that shortens the test time and leads the test subject to focus on the test during the test may be required.

The information collection method in the utterance approach generally requires a method for the utterer to obtain a question for utterance to make an utterance and a providing device and a device for storing utterance information when making an utterance.

In general, in a language test, the question is heard through a smart device and, if necessary, the content for the corresponding question is viewed through the screen of the smart device while answering. There may be several types of such smart devices. For example, it may be a tablet, a smartphone, a notebook, a mobile robot, or the like capable of mobile support. Alternatively, there may be a personal PC or the like as a stationary type.

### [Utterance data collection method using smart device and wearable device and analysis method using cloud and AI]

Embodiments of the disclosure provide a method of performing a cognitive impairment or language test through an app or a web through a wearable device and a smart device. There is also proposed a method for selecting mild cognitive impairment (MCI) and early dementia by utilizing cloud and converging AI technology for the test. Through this method, tests may not only be performed at senior citizen centers, dementia relief centers, and welfare centers for the elderly, but smart devices owned by individuals may also be used conveniently, easily, and periodically in daily living spaces where the elderly actually reside.

In particular, embodiments of the disclosure use a wearable device capable of wirelessly listening to a question performed on a web or an app of a smart device, receiving an answer speech to the heard question, and transferring the answer speech to the smart device without distortion. The answer speech transferred from the wearable device to the smart device is stored in the smart device, and the stored speech is transferred to the cloud.

As another example, while the question is being asked, the speech collected through the microphone of the wearable device is directly stored in the wearable device, and the speech data is transferred at once.

Labeling may also be easily processed by listening to questions and storing answers through the web or app of the smart device through the wearable device. This is because questions and answers may be stored consistently. Further, such a method may also be applied in which answers may be recorded using acceleration information in the case of choice questions with a gyroscope or acceleration sensor embedded. In particular, the time of the answer to the question is important, and information about ending and maintaining the answer may be provided to obtain more accurate information about the answer utterance.

The proposed method builds standardized utterance big data for the elderly in Korea, and based on this, learns about the linguistic characteristics of subjects for MCI and early dementia symptoms. Further, it is possible to extract linguistic features that change over time by accumulating years of test data for the same person in big data. It is preferable that the learned AI classifier presents normal and abnormal degrees for cognitive impairment. For example, it is possible to suggest how far it is from the average value of normal elderly people. Further, based on utterance data extracted from data of patients with MCI, it is possible to present how far the current state of MCI deviates from the existing accumulated data. In other words, as the assessment result of the language test, the assessments for each individual question are converted into scores and displayed, and the scores for each question may differ depending on the type of the task for leading to utterance. Further, as an example of the score for each question, a weight or importance score for linguistic features such as the number of used words, the frequency, the composition of a sentence, the utterance start time after a question, etc. may be prepared in advance, and it is applied to the collected utterance data so that the utterance data for the question is quantized as the sum of the weight-applied linguistic features.

In the speech test method, it is possible to choose a method of speaking within a limited time for a given topic. It is also possible with speech, video, and text presenting a given topic. There are various topics (tasks) presented, such as waiting for family names, waiting for animal names, waiting for place names, waiting for country names, etc. Any topic word may be selected as long as the number of the ones answered in a short answer may be counted. Therefore, for not only nouns, but also predicates such as 'is beautiful,' 'is pretty,' 'is lovely,' etc., or verbs such as run or flee, the number of words may be counted. Accordingly, any topic that may lead to a number of related words or may be uttered is possible.

The limited time is limited to about several minutes for general topic words. However, it is preferable to be able to utter for at least 30 seconds, and to design to finish utterance within three minutes for one topic word.

As a simple assessment method, the cognitive function is assessed by simply assessing the number of words for each topic word, the difficulty level of the word, and the number of repetitions of the same word.

Further, it is possible to enhance the accuracy of the cognitive function by assessing each of two or more topic words rather than one topic word. In other words, by performing on each of the plurality of topic words, the assessment result for one topic word and the assessment result for the second or third topic word each may be presented, and the three results may be comprehensively assessed.

Tasks include understanding tests, expression/speaking tests, and memory tests, and there are various questions in each task. As a quantification method, utterance data obtained for questions used in the task may be quantified and an average of the quantified utterance data for all tasks may be obtained. Further, the test results may be quantified independently for each task.

If each test result is quantified as described above, the utterance data can be translated as the linguistic data collected from a prepared linguistic test performed on a normal person, a patient diagnosed with MCI, a dementia symptom, etc., and then transcribed, and data indicating linguistic features (word frequency, utterance time after questioning, used words, interjections, sentence structure, etc.) derived..

In particular, the current language test has various tasks, and there are several questions corresponding to the task for each task. For example, language tests include comprehension tasks, expression tasks, and memory tasks. Further, there are various questions in the expression task, such as explaining pictures and telling fairy tales. In the memory task, several words about animals or fruits each are called, and a test to say words that come to mind is performed on each thereof. Questions belonging to each task include questions with similar difficulty and questions with high difficulty. However, depending on the actual subject (utterer), it may not be necessary to ask similar questions in all the tasks. To solve this problem, it is preferable to select the optimal question for each task. For the method of selecting these optimal tasks or questions, it may be extracted based on language test big data. For example, the difficulty level of the task may vary depending on the subject's condition. Alternatively, tasks having duplicate features may be optimized and used. So far, not much academic research has been made on the question set considering the correlation between tasks. For this optimal question set, features of correlations between questions according to gender, age, educational background, etc. of subjects may be extracted through artificial intelligence learning based on big data collected through almost all the tasks and questions about each task, as in the currently used method. For example, a question set of a task suitable for the difficulty level of the picture description according to the educational background may be selected.

As another method, there are prepared question set of a task, and next questions may be selected based on information about previous questions and answers. Information and answers to previous questions are analyzed in real time. The analysis includes the utterer's understanding of the questions, answer ability, and linguistic features. Therefore, selection of the next question is based on basic information about the utterer's gender, age, educational background, etc. and analysis results of previous questions. In order to select the next question, an artificial intelligence technology may be applied to create an appropriate model.

The optimal task set may be so selected (selection method may be used utilizing an AI model for selecting the optimal task set based on the big data for the existing subjects) to proceed with the test. This may shorten the test time and allow the elderly to be easily tested.

The optimal task list should be able to be determined upon each individual question task in order to obtain utterance information for each task.

### [Definition of function for wearable device]

As mentioned above, in the speech reception method using a microphone mounted on a smart device, a certaindistance is present between the smart device and the utterer, so that ambient noise and other people's speeches are added to the utterer's utterance. The accuracy of the linguistic and acoustic analysis results using the input utterance deteriorates.

Embodiments of the disclosure may provide a method for utilizing a wearable device for precisely collecting utterance data. It is preferable that the proposed wearable device is wirelessly connected to the smart device. Further, a separate app/web for connecting the wearable device and the smart device may be used.

A wearable utterance-collection device for the elderly may include noise removal technology to eliminate external noise. The noise removal technology not only increases the speech recognition rate of the utterer by directly removing various life noises, irregular noises, etc., but also removes unnecessary signals in the method of analyzing speech features, so that the features of the speech itself may be more clearly identified and analyzed, and the language test may be easily performed even in the space with ambient noise.

The speech signal processing performed by the wearable device to increase the speech recognition rate of the AI speech recognition device and to secure as much speech information from the utterer as possible may include, e.g., two functions.

First, there may be a function of removing noise depending on the environment. For example, when life noise other than the utterer or speeches other than the utterer's speech are introduced from the ambient environment, it may be necessary to remove it. Accordingly, it is possible to increase the speech recognition rate of the utterer in the final assessment stage.

Second, when the magnitude of the utterer's speech is small, it is necessary to increase it. Further, a small speech among the contents uttered by the utterer needs to be further found in the final speech recognition process. When a person listens to and transcribes it, it may be heard in detail thanks to the transcription, but in everyday life, recognition by an artificial intelligence speech recognition device using utterance information by the utterer may be very different. In other words, all of the self-talk, interjection, etc. included in the middle may be excluded by the AI speech recognition device due to its low magnitude, and in this case, information necessary for analysis may be lost. Therefore, in order to secure utterance data displayed small in addition to the speech accurately uttered by the wearable device, it is necessary to increase the speech recognition rate in the AI speech recognition device by amplifying the corresponding speech. Further, since noise is also amplified during amplification, it may be preferable that noise removal and amplification proceed at the same time.

The noise removal process is a method of changing the sound signal input through the microphone into the frequency domain and removing noise power in the frequency domain.

As a method of removing noise, the utterer's speech is pre-learned and stored. In this case, the utterer's acoustic features, i.e., the frequency features and magnitude information about the speech, may be extracted and utilized. A method of removing all of the speeches or life noises having information other than the secured speech features of the utterer may be used. To remove noise, the utterance data input in real time may be converted into the frequency domain, and the life noise and speech information absent in the utterer's features may be removed by utilizing the speech information about the utterer already stored.

Another method is to use a classifier or detector capable of classifying noise and speech environments. In other words, if noise and speech may be efficiently separated, incorrect speech recognition may be avoided, and noise power may be removed in frequency analysis of speech. Accordingly, by enhancing the signal-to-noise ratio (SNR), it is possible to listen more clearly and cleanly with lower noise. Further, a method of removing data other than the utterer's speech from utterance data input through the microphone may be used using an intelligent classifier through learning about traffic noise, life noise, or the like.

Further, various noise removal techniques may be applied. In particular, various methods of removing noise from a frequency band by converting an input signal into a frequency band may be applied.

The wearable device may have a receiver (speaker) capable of hearing a task question for utterance from the smart device. It is preferable that the speaker is inserted into the ear. Further, it may be manufactured in a bone conduction type. Through this, when a task question is heard using only the smart device, it may be heard with external noise, and thus the task question may not be heard well, but the question may be heard much more accurately using the proposed method, which is advantageous for test.

It is advantageous that the microphone of the wearable device is positioned near where the subject utters. For example, it may be a neckband type utterance collector or an earbud type. A lightweight wearable device is used. This device allows direct collection of utterance information about the subject as possible.

The wearable device may use a directional microphone. It is possible to more clearly receive only the speech from the utterer's mouth using the directional microphone.

Further, since the sound quality itself may be distorted and the speech recognition rate may be lowered when the speech is transmitted, digitized data is transmitted rather than transmitting it by speech from the wearable device to a smart device. It may be converted into audio by the smart device..

The wearable device may selectively amplify the speech received through the microphone. For example, a specific frequency band, a high frequency band, a low frequency band, or the like may be selectively amplified.. The features of the speech received through the microphone are partially changed according to the features of the microphone or in an analog-to-digital converter (ADC) that converts analog into a digital signal. The digitized sound or data may be wirelessly transferred to the smart device with a small signal in a partial frequency band removed according to the characteristics of the signal-to-noise ratio according to the wireless communication characteristics. For example, elderly people with cognitive impairment may mutter in a low tone when they do not think of an answer to a question or when they think of an answer to a question. The information may be used as an important item in linguistic feature analysis, so it is necessary to secure the original data of the utterer as possible. Or, in specific expressions, if the sound is small, the answer to the question may not be recognized, resulting in a wrong result of assessment. In another case, the speech magnitude of the elderly itself may be small, and the speech signal input to the smart device may be small.

In general, as the signal input from the microphone is digitized, low sounds all may be deleted in the device feature and speech signal processing process. Even if deleted, there is no big problem in communicating in daily life, but various information may be lost in linguistic and acoustic features. When a low sound is deleted, the final assessment result may be wrong because it is not included in the assessment result, even though it must be reflected in the assessment score.

Therefore, such issues may be addressed by using a method of amplifying low sounds while refraining from amplifying high sounds for the utterance data received through the microphone. Further, by selectively amplifying the input sound according to the frequency through frequency band analysis, the degree of speech recognition may be enhanced, expressions having acoustic features may not be removed, and low sounds may be amplified to allow for accurate assessment using utterance data.

There are provided advantages of being able to transfer the original speech signal of the utterer without distortion by amplification in the wearable device and then analyze it in a server or a smart device. In other words, selective amplification of the sound received from the wearable device may be performed in a server having very good computing power, but there may be a problem that a lot of linguistic and acoustic information is deleted while being converted into digital data and being transferred to the server.

The wearable device may directly store answers to individual task questions in the wearable device, and may bundle multiple answers and transmit them to the smart device. Further, the utterance data received for each question may be wirelessly transferred to the smart device before starting the next question.

### [Definition of functions for app/web]

The utterance collection app/web should include the following functions. When performing a task, it is necessary to be able to transfer a speech on the instructions for the task to the wearable device. Further, it should come up with a function of being able to given assistance when the test is done wrong due to a mistake or error by monitoring the process performed by the older people through the smart device. In general, if an error occurs during the test through an app/web, or if a re-test is performed, the test may be performed again. Further, when a wrong button is pressed during the test, causing the test to stop, the related issues should be able to be addressed with the aid of the tester administrative or the app/web administrative.

The utterance data for each question may be synchronized and stored. Digitized data stored in the smart device is sent to the cloud according to a set protocol.

Responses to each question in a task sent to the cloud are subjected to determination for MCI, dementia symptoms, and normal conditions through a model that has already been trained for the diseases. This determination may also be made with a score, and it may be indicated by how dangerous it is compared to the normal standard. For example, if a high-risk result is found, it may be associated with pre-treatment through a thorough screening test or diagnostic test at a hospital.

As such, the cloud-based assessment method may derive reliable results using a lot of data, but utterance data should be directly transmitted to the cloud and requires a cloud. Therefore, a method of providing a service with only a wearable device even in an environment in which the cloud may not be used is proposed.

This is the case when a MCI or cognitive impairment classifier based on utterance data is embedded in a wearable device and used. In other words, through the smart device, the utterance proceeds while looking at and listening to task questions. The utterance data is input through the microphone of the wearable device, and noise removal as necessary and speech amplification as necessary are performed. The wearable device is equipped with a cognitive impairment classifier that has already been trained, and the cognitive impairment is early selected using utterance data input to the microphone through the classifier. It is preferable that the classifier used at this time uses smaller linguistic and volume features than the cloud-based classifier considering the computing ability and storage ability of the wearable device.

As a method for collecting utterance data, various tasks may be automatically stored to make labeling easier.

For example, a method of passing over if there is no speech for a predetermined time or longer, a method of recording an answer using physical sensor information in the case of a choice question by embedding a gyroscope or acceleration sensor, a method of specifically interacting with a device (for example, a method through a command, a method through a question such as 'next' or 'shall we go next?', a method of passing over by putting a specific physical function on the device (touch type, button type, etc.) may be used. Further, in order to lead to concentration on task progress, a message for calling attention may be output every predetermined time. The content and form of the message may vary, and for example, the name of the test subject may be called, or a comment such as "Focus on the test" may be output in the form of a sound. Alternatively, it is possible to output light or an image that may easily lead to focus attention, such as a method of blinking light.

This suggested methods not only make labeling easier when storing data, but also makes it easier to separate each task, and make it possible to select an optimal question set for each task by subdividing various questions included in the task. Through this, the selection time may also be greatly shortened.

Such a wearable device may be used only to store the utterer's utterance and transfer the utterer's utterance to the smart device. In other words, the utterer may directly listen to the question and see the question through the smart device. The wearable device collects, stores, and transfers utterances for each question to the smart device. Wireless communication is preferable for connection between the wearable device and the smart device, and utterance data collected through the microphone in the wearable device may be converted into digital data through the ADC, transmitted as digital data, or converted back into speech to transmit speech. Even in this process, noise or speeches other than the utterer's speech are removed to facilitate speech recognition and analysis of the utterer.

### [About service methods]

Scenarios for service methods are as follows.

The utterer is wearing the wearable device, and may view or hear questions about the test through an app of the smart device or a PC web. Through the wearable device, the test is conducted while listening to and looking at the test questions conducted in the app of the smart device. Among the questions, the utterer's speeches are collected through the microphone of the wearable device and transferred to the smart device as digital data or utterance data. The smart device transmits the utterer's answer to each question to the cloud, and the cloud has an assessment model for each answer, thereby scoring the answer to each question. The assessment and analysis of each answer are performed through a separate speech recognizer and a normal or mild cognitive impairment classifier. In other words, the input utterance data is converted into text through the speech recognizer. Sentences converted into text are analyzed and assessed according to a predetermined rule. The classifier learns and stores linguistic and acoustic features of normal people and MCI patients using answers to questions already presented. When the answer to the entire test is completed, the scores are summed up to provide the utterer with how far the degree of MCI and depression is from the standard normal person.

As another method, a speech recognizer and a classifier capable of determining MCI, depression, etc. may be used in the wearable device. In other words, the subject may wear the wearable device, look at the smart device while seeing, hearing, and answering the questions. In particular, the utterance data is stored through the microphone of the wearable device. The wearable device has a built-in speech recognizer and classifier. The speech recognizer converts the input utterance data into text. Sentences converted into text are analyzed and assessed according to a predetermined rule. In this case, it is preferable for the wearable device to know the question information about the utterer, but it may be possible even without knowing the questions. The wearable device is equipped with a classifier that learns linguistic and acoustic features of normal people and MCI patients for questions already presented. This method does not use the cloud, so the utterer's test results may be quickly and immediately checked. Further, it is not required to send the one's speech data to the cloud.

Examples in which the above-described embodiments of the disclosure may be implemented are described with reference to the accompanying drawings.

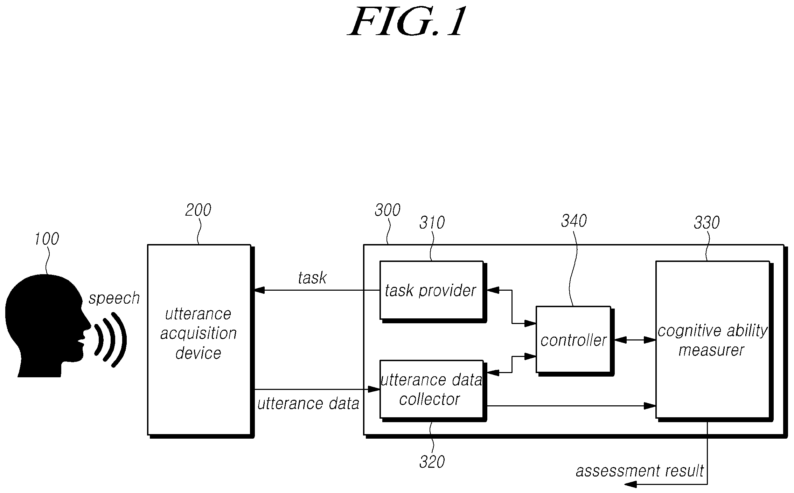

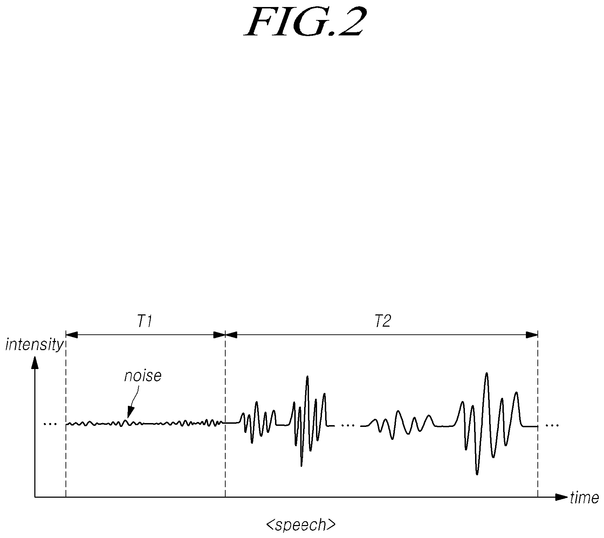

FIG. 1 is a view schematically illustrating an example of a configuration of a cognitive ability assessment system according to embodiments of the disclosure. FIGS. 2 to 5 are views illustrating examples of a method of performing a cognitive ability assessment by a cognitive ability assessment system according to embodiments of the disclosure;

Referring to FIG. 1, a cognitive ability assessment system according to embodiments of the disclosure may include, e.g., an utterance acquisition device 200 and a cognitive ability assessment device 300.

The utterance acquisition device 200 may obtain a speech by the utterance of the user (or utterer) 100. The utterance acquisition device 200 may output a question for leading to the utterance of the user 100.

The utterance acquisition device 200 may be implemented in a wearable form or a portyable AI speaker form, but is not limited thereto.

The cognitive ability assessment device 300 may communicate with the utterance acquisition device 200 in a wired or wireless manner, and provide a task provided to the user 100 to the utterance acquisition device 200. Further, the cognitive ability assessment device 300 may receive utterance data based on the speech of the user 100 obtained by the utterance acquisition device 200.

FIG. 1 exemplarily illustrates a structure in which the cognitive ability assessment device 300 performs assessment on the user 100 through the utterance acquisition device 200. In some cases, the cognitive ability assessment device 300 may directly provide a task to the user 100 and receive utterance data of the user 100 without the utterance acquisition device.

The cognitive ability assessment device 300 may be implemented in the form of a movable terminal or a server, but is not limited thereto. In some cases, some components of the cognitive ability assessment device 300 may be included in the terminal, and the other components may be included in the server.

The cognitive ability assessment device 300 may include, e.g., a task (or a question) provider 310, an utterance data collector 320, a cognitive ability measurer 330, and a controller 340.

The task provider 310 may provide a task for assessing the user's cognitive ability. The utterance data collector 320 may obtain utterance data according to the user's answer to a task. The cognitive ability measurer 330 may produce an assessment result of the cognitive ability of the user 100 based on the utterance data. The controller 340 may control the operation of each component included in the cognitive ability assessment device 300.

The task provider 310 may select a task to be provided to the user 100 during a test period for assessing the cognitive ability of the user 100 and provide the selected task to the user 100 through the utterance acquisition device 200.

The task provider 310 may differently select a task to be provided to the user 100 based on personal information such as educational background, gender, age, etc. of the user 100.

The task provider 310 may provide a question generated based on information recognized by the user 100 as a task. The information recognized by the user 100 may be information personally recognized by the user 100 or information commonly recognized by the general public.

Information personally recognized by the user 100 may be obtained through a device used by the user 100, such as a smart device. Information commonly recognized by the general public may be, e.g., a widely known fairy tale. Commonly recognized information may be used to generate a task after identifying whether the user 100 recognizes it in advance.

The task provider 310 may generate a task including a question for assessing the cognitive ability of the user 100.

The task provider 310 may provide basic questions used to assess the cognitive ability of the user 100. The task provider 310 may provide the user 100 with questions requesting a plurality of determinations at the same time. For example, the task provider 310 may provide a task of simultaneously determining two or more elements (features) in order to solve the task through content expressed together through pictures, colors, or the like in addition to the content of the sentence.

The task provider 310 may change some content from the information recognized by the user 100 and provide the changed content to the user 100, and then provide the user 100 with a question generated from the changed recognition information. For example, the task provider 310 may change some words in the information recognized by the user 100. The task provider 310 may change the words corresponding to a specific part of speech (e.g., a noun, a pronoun, a preposition, etc.). The task provider 310 may provide the user 100 with a question based on information in which the content is slightly changed from the information recognized by the user 100, thereby identifying whether the user 100 recognizes the changed content and gives an accurate answer.

The task provider 310 may provide the question through the speech to the user 100. In some cases, the task provider 310 may provide an image displayed on the screen or a task requiring an action of the user 100.

The task provider 310 may lead to the utterance of the user 100 through the provided task.

The task provider 310 may provide a task of leading to a specific utterance of the user 100. For example, the task provider 310 may provide a task of allowing the user 100 to pronounce a voiced sound or a plosive. The task provider 310 may provide a task of repeating a specific utterance for a predetermined period of time. As described above, in the case of a normal person and a MCI person, various tasks capable of leading to a distinct pronunciation may be provided to the user 100.

Further, the task provider 310 may adjust the task provided later according to the answer of the user 100. By adjusting the task according to the answer of the user 100, the utterance of the user 100 may be continuously led to, and utterance data for assessing cognitive ability may be obtained. Further, in order to maintain the concentration level of the user 100 on the task, the task provider 310 may output a sound through the utterance acquisition device 200, an image through a smart device, or the like that leads to the concentration of the user 100 every predetermined time while the task is provided. Accordingly, the concentration level of the user 100 may be maintained at a predetermined level or higher during the time when the task is performed.

If an utterance is generated according to the answer of the user 100 to the task, the speech by the utterance may be obtained by the utterance data collector 320.

The utterance data collector 320 may receive the utterance data obtained from the user through the utterance acquisition device 200.

If the speech by the utterance of the user 100 is obtained, the utterance acquisition device 200 may convert the speech into utterance data in a digital form. The utterance acquisition device 200 may convert the obtained speech into utterance data in a digital form as it is and transfer the same to the utterance data collector 320.

Alternatively, the utterance acquisition device 200 may adjust the obtained speech to a form capable of enhancing the performance of the cognitive ability assessment and then convert the same into utterance data in a digital form. For example, the utterance acquisition device 200 may remove noise from the obtained speech and then convert it into utterance data. As another example, the utterance acquisition device 200 may amplify at least a portion of the obtained speech and then convert the same into utterance data.

Noise removal or speech amplification may be performed separately during a period in which a task is provided by the task provider 310 and an answer to the task of the user 100 is made.

For example, referring to FIG. 2, the task provider 310 may provide a task during a test period divided into a first test period T1 and a second test period T2.

The first test period T1 and the second test period T2 may be periods that are temporally divided. The first test period T1 may be a period before the second test period T2.

The first test period T1 may be, e.g., a period during which a preliminary task for the main test is performed before the main test for cognitive ability assessment is performed. The second test period T2 may be, e.g., a period during which the main test for cognitive ability assessment is performed.

The first test period T1 may be, e.g., a period for providing guidance on the test before performing the test in the second test period T2. Alternatively, the first test period T1 may be a period during which a preliminary test is performed prior to the second test period T2. Alternatively, the first test period T1 may be a period in which a test for cognitive ability assessment and a test or task for another purpose are simultaneously performed.

For example, guidance for the present test may be provided during the first test period T1. When guidance is provided during the first test period T1, the speech by the utterance of the user 100 may not be generated.

The utterance acquisition device 200 may obtain noise generated around the user 100 during the first test period T1. The utterance acquisition device 200 may analyze the frequency band of the noise obtained during the first test period T1. The utterance acquisition device 200 may remove noise generated during the second test period T2 when the main test is performed based on the frequency band of the noise obtained during the first test period T1.

For example, the main test for cognitive ability assessment may be performed during the second test period T2. The user 100 may give an answer to the task provided according to the main test during the second test period T2. The utterance acquisition device 200 may obtain the speech by the utterance according to the answer of the user 100 during the second test period T2. During the second test period T2, a sound obtained by mixing the speech of the user 100 and ambient noise may be obtained.

Since the utterance acquisition device 200 has identified the frequency band of the noise around the user 100 during the first test period T1, the utterance acquisition device 200 may remove the noise from the sound obtained during the second test period T2 by analyzing the frequency band of the sound obtained during the second test period T2. The utterance acquisition device 200 may convert the noise-removed speech (utterance in an analog data) into utterance data in a digital form or digital data and transfer the same to the utterance data collector 320. The cognitive ability assessment device 300 may obtain utterance data based on the speech from which noise is removed by an external device.

Further, the utterance acquisition device 200 may adjust the magnitude of at least a portion of the speech of the user 100 obtained during the second test period T2 and then convert the same into utterance data.

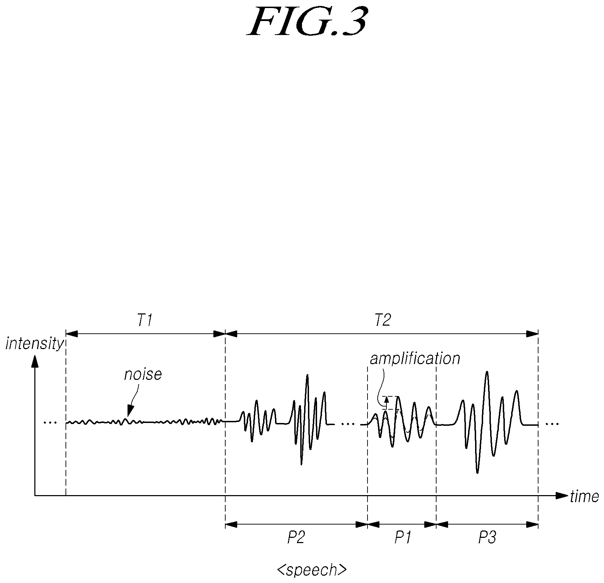

Referring to FIG. 3, ambient noise of the user 100 may be obtained during the first test period T1. The utterance acquisition device 200 may identify the frequency band of the noise based on the noise obtained during the first test period T1.

The speech of the user 100 may be obtained during the second test period T2. Noise may be removed from the sound obtained during the second test period T2 based on the frequency band of the noise identified during the first test period T1.

The utterance acquisition device 200 may adjust the magnitude of at least a portion of the speech before converting the speech of the user 100 obtained during the second test period T2 into digital utterance data.

For example, when the speech of the user 100 obtained during the second test period T2 is referred to as a basic speech, the utterance acquisition device 200 may generate a test speech in which the magnitude of at least a portion of the basic speech is adjusted. The utterance acquisition device 200 may amplify, e.g., the magnitude of the first portion P1 of the basic speech. The utterance acquisition device 200 may not amplify the magnitude of the second portion P2 or the third portion P3, which is a portion other than the first portion P1 of the basic speech.

The cognitive ability assessment system according to embodiments of the disclosure may assess the cognitive ability of the user 100 through utterance analysis of the user 100. A small speech that may be removed by noise may also be required for utterance analysis. Since the noise is removed from the sound obtained in the second test period T2 based on the frequency band of the noise analyzed during the first test period T1, the remaining sound may be viewed as the speech of the user 100. By amplifying the magnitude of a portion of the speech of the user 100, analysis using utterance data based on the overall speech of the user 100 obtained during the second test period T2 may be performed.

The utterance acquisition device 200 may amplify the speech of a portion of the user's speech obtained during the second test period T2 in which the speech magnitude is less than or equal to a predetermined magnitude. Alternatively, the utterance acquisition device 200 may amplify the speech of the portion in which the length of the portion in which the speech magnitude is less than or equal to the predetermined magnitude is larger than or equal to the predetermined length. An example in which the utterance acquisition device 200 amplifies a portion of the speech of the user 100 is not limited thereto. All of the methods of amplifying the magnitude of at least a portion of the speech of the user 100 obtained during the second test period T2 to enable utterance analysis by the cognitive ability assessment device 300 may be included in embodiments of the disclosure.

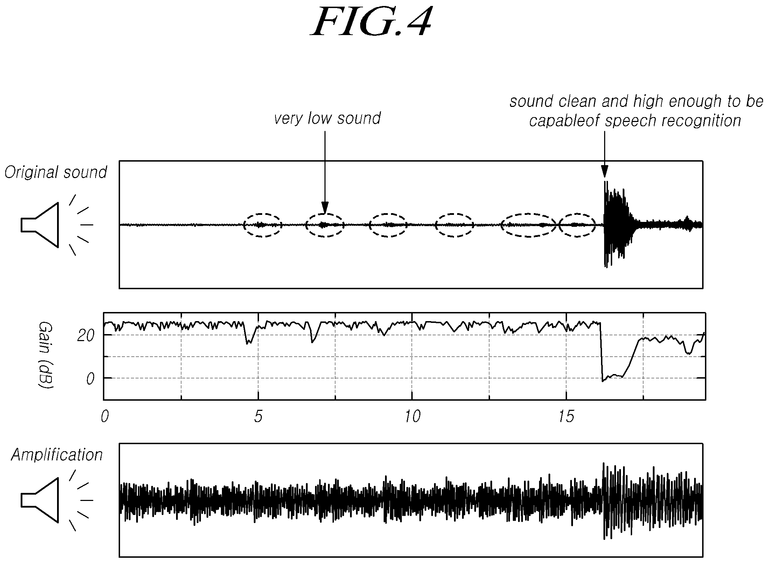

For example, FIG. 4 exemplarily illustrates a compression amplification technique (WDRC). FIG. 4 illustrates an example of partially amplifying and providing an original sound. In FIG. 4, the horizontal axis represents the time, and the vertical axis represents the magnitude of speech.

As illustrated in FIG. 4, the original sound may include a very low sound portion and a sound portion clean and large enough to be capable of speech recognition. The very low sound may be heard in the first half and the high sound may be heard in the last part. In everyday life, small speeches such as in the first half may not be recognized by the actual speech recognizer and may not be used as information.

To compensate for this, e.g., a different gain may be applied according to a portion of the speech to partially amplify the speech. As illustrated in FIG. 4, a gain of about 25 dB may be given to the small-sound section, and amplification may be rarely performed in the large-sound section.

The high sound is recognized as it is, and the low sound is amplified to an appropriate magnitude so that speech recognition may be performed well. The amplification method may simply amplify the entire speech, or may select and amplify a specific frequency band.

In this way, the low sound may be heard by amplification. In particular, if noise is amplified together and is removed together, the sound that has not been actually heard may be heard, and necessary information to be obtained through the speech may be sufficiently secured.

As described above, the utterance acquisition device 200 may provide the user 100 with a preparation time for the main test through the first test period T1, and at the same time, perform a process capable of removing noise from the speech of the user 100.

Further, depending on cases, the speech by the utterance of the user 100 may be obtained even during the first test period T1. The utterance data according to the speech of the user 100 obtained during the first test period T1 may or may not be used to assess the cognitive ability of the user 100.

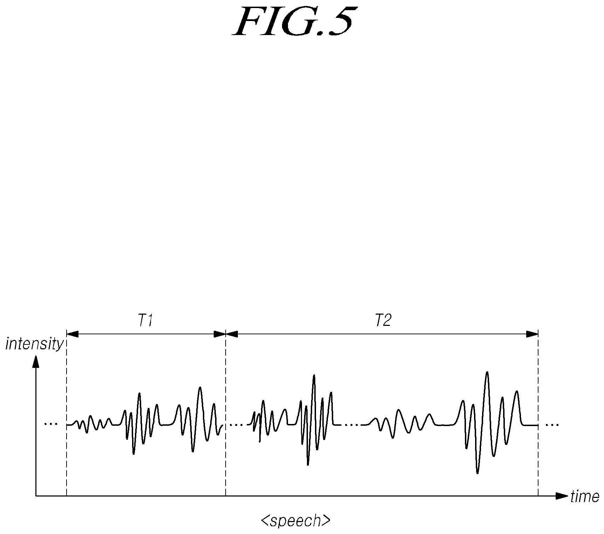

Referring to FIG. 5, guidance for the main test may be provided to the user 100 or a preliminary test may be provided during the first test period T1. According to the case, whether to perform the main test or the difficulty level of the main test may be determined in the second test period T2 according to the answer of the user 100 obtained in the first test period T1.

The speech by the utterance of the user 100 according to the task provided during the first test period T1 may be obtained.

The utterance acquisition device 200 may generate first utterance data based on the speech obtained during the first test period T1. The utterance acquisition device 200 may generate second utterance data based on the speech obtained during the second test period T2.

The utterance acquisition device 200 may transfer the first utterance data and the second utterance data to the utterance data collector 320.

The cognitive ability assessment device 300 may produce the assessment result regarding the cognitive ability of the user 100 based on the second utterance data based on the speech obtained during the second test period T2. The cognitive ability assessment device 300 may use the first utterance data based on the speech obtained during the first test period T1 to produce the assessment result regarding the cognitive ability of the user 100.

For example, when data required to produce the assessment result is insufficient in the process of producing the assessment result regarding the cognitive ability of the user 100 based on the second utterance data, the cognitive ability assessment device 300 may produce the assessment result regarding the cognitive ability of the user 100 including the first utterance data.

Alternatively, the cognitive ability assessment device 300 may produce the assessment result regarding the cognitive ability of the user 100 by including both the first utterance data and the second utterance data.

When producing the assessment result regarding the cognitive ability of the user 100, the cognitive ability assessment device 300 may reflect the first utterance data and the second utterance data at the same level.

Alternatively, in some cases, the first test period T1 may be viewed as a period in which the reliability of the test result for the user 100 is lower than the second test period T2, and thus the assessment result regarding the cognitive ability may be produced with different weights applied to the first utterance data and the second utterance data. For example, a first weight may be applied to the first utterance data and a second weight may be applied to the second utterance data to produce the assessment result regarding the cognitive ability of the user 100. The first weight may be smaller than the second weight.

As described above, the utterance data based on the speech by the utterance of the user 100 obtained during the first test period T1 may also be used to produce the assessment result regarding the cognitive ability.

Further, even when the speech by the utterance of the user 100 is obtained in both the first test period T1 and the second test period T2, the utterance acquisition device 200 may perform a process of removing noise from the speech of the user 100 obtained in each test period or adjusting the magnitude of the speech.

For example, the utterance acquisition device 200 may obtain the speech by the utterance of the user 100 during the first test period T1. The utterance acquisition device 200 may identify the frequency band of the speech of the user 100 by analyzing the frequency band of the speech of the user 100 obtained during the first test period T1.

The utterance acquisition device 200 may remove noise corresponding to a frequency band other than the frequency band of the speech of the user 100 from the sound obtained during the first test period T1 and the second test period T2. The utterance acquisition device 200 may amplify the magnitude of a portion of the noise-removed speech of the user 100.

Accordingly, even when the speech by the utterance of the user 100 is obtained during the first test period T1 and the second test period T2, utterance data based on the speech where the noise is removed and a portion of the noise is amplified may be provided.

The utterance data transferred by the utterance acquisition device 200 may be provided to the cognitive ability measurer or analyzer 330.

The cognitive ability measurer (analyzer) 330 may produce the assessment result regarding the cognitive ability of the user 100 by analyzing the utterance data.

The cognitive ability measurer 330 may produce the assessment result regarding the cognitive ability of the user 100 using at least one of various elements (or features) included in the utterance data of the user 100.

For example, the cognitive ability measurer 330 may produce the assessment result regarding the cognitive ability of the user 100 based on the number of words used by the user 100 during a predetermined period included in the utterance data. The cognitive ability measurer 330 may produce the assessment result regarding the cognitive ability of the user 100 based on the number of words used in each of the plurality of sections included in a predetermined period.

The cognitive ability measurer 330 may produce the assessment result regarding the cognitive ability of the user 100 based on the type of the word used by the user 100 for a predetermined period included in the utterance data. The type of the word may mean the part of speech of the word. The assessment result regarding the cognitive ability of the user 100 may be produced based on the frequency at which words of a specific part of speech are used.

The cognitive ability measurer 330 may produce the assessment result regarding the cognitive ability of the user 100 based on the number of duplicate words used by the user 100 during a predetermined period included in the utterance data. The number of duplicate words may be divided and counted for each of the plurality of sections included in the preset period. The assessment result regarding the cognitive ability of the user 100 may be produced based on the number of duplicate words used in each section.

The cognitive ability measurer 330 may produce the assessment result regarding the cognitive ability of the user 100 based on the number of words used by the user 100 for each section of the predetermined period included in the utterance data.

The cognitive ability measurer 330 may produce the assessment result regarding the cognitive ability of the user 100 based on the utterance period and the pause period included in the predetermined period among the utterance data. The pause period may refer to a period during which an utterance is not generated. Alternatively, the pause period may refer to a period of a predetermined time or more among periods between utterance periods.

The cognitive ability measurer 330 may produce the assessment result regarding the cognitive ability of the user 100 based on a change rate of the length of at least one of the utterance period or the pause period included in the predetermined period among the utterance data. For example, the cognitive ability measurer 330 may produce the assessment result regarding the cognitive ability of the user 100 based on a change rate of the length of the pause period. Further, the assessment result regarding the cognitive ability of the user 100 may be produced based on the rate of change of the length of the pause period in each of the plurality of sections included in the predetermined period.

The cognitive ability measurer 330 may produce the assessment result regarding the cognitive ability of the user 100 based on the amount of change in the ratio between the utterance period and the pause period included in the predetermined period among the utterance data. The ratio between the utterance period and the pause period may mean a ratio between the two in one section in which the utterance period and the pause period are repeated. Alternatively, it may also mean a ratio between the utterance period and the pause period in each of the plurality of sections included in the predetermined period. The assessment result regarding the cognitive ability of the user 100 may be produced based on the uniformity level of the proportion of the pause period.

The cognitive ability measurer 330 may produce the assessment result regarding the cognitive ability of the user 100 based on one or a combination of two or more of the above-described examples.

The cognitive ability measurer 330 may provide the user with the assessment result regarding the cognitive ability of the user 100.

The cognitive ability measurer 330 may present the assessment result regarding the cognitive ability as a score, may divide the same into two or more grades and present them, or may provide the same as whether to give a warning regarding the MCI.

The cognitive ability measurer 330 may compare the analysis result of the utterance data of the user 100 with the past data of the user 100, and produce the assessment result of the cognitive ability of the user 100. Alternatively, the cognitive ability measurer 330 may compare the analysis result of the utterance data of the user 100 with average data of other users other than the user 100, and produce the assessment result of the cognitive ability of the user 100. Alternatively, the cognitive ability measurer 330 may produce the assessment result regarding the cognitive ability of the user 100 by comparing the result of analysis of the utterance data of the user 100 with data derived from patients with MCI or dementia. In some cases, the cognitive ability measurer 330 may produce the assessment result regarding the cognitive ability of the user 100 by combining two or more of the above-described comparison methods.

As described above, according to embodiments of the disclosure, the utterance acquisition device 200 and the cognitive ability assessment device 300 may easily assess the cognitive ability of the user 100, and if there is a risk of MCI, provide corresponding information to the user 100 to detect MCI or dementia early.

As described above, the cognitive ability assessment device 300 may be implemented in various forms such as a mobile or PC terminal or a cloud server, and in some cases, the components of the cognitive ability assessment device 300 may be distributed to the terminal and the server to configure a cognitive ability assessment system.

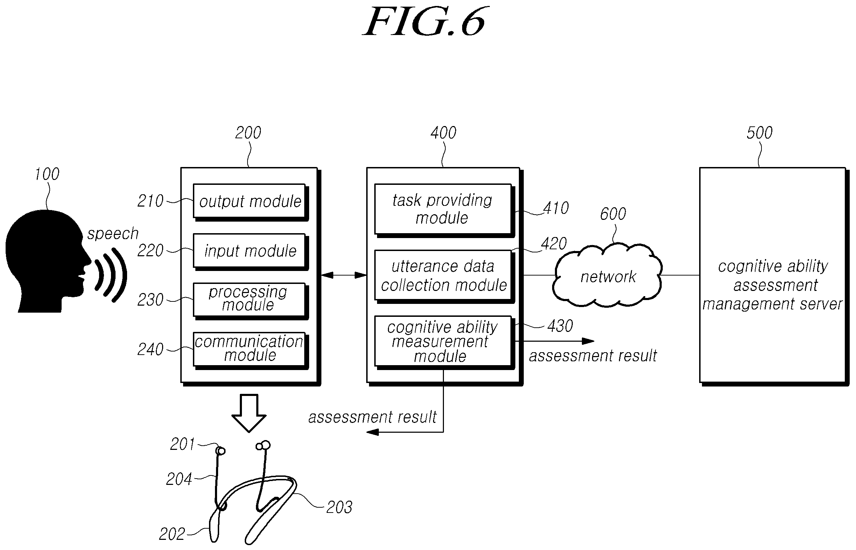

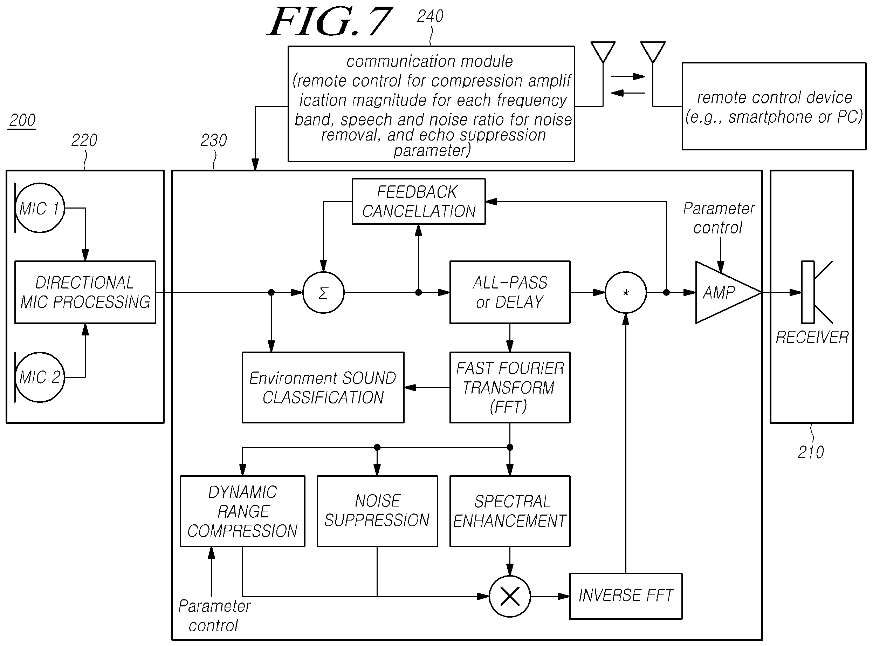

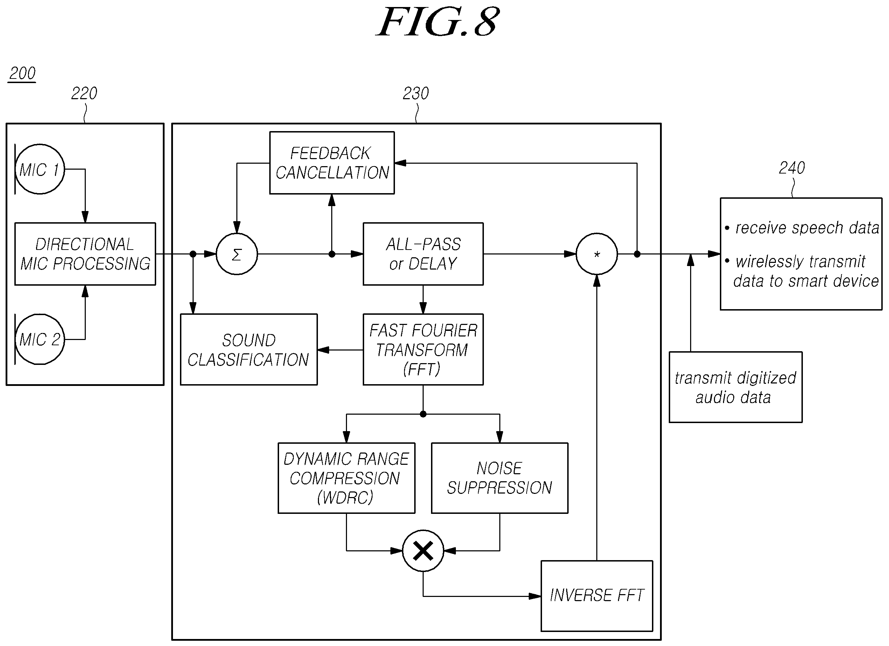

FIG. 6 is a view schematically illustrating another example of a configuration of a cognitive ability assessment system according to embodiments of the disclosure. FIGS. 7 and 8 are views exemplarily illustrating a configuration of the utterance acquisition device 200 illustrated in FIG. 6.

Referring to FIG. 6, a cognitive ability assessment system according to embodiments of the disclosure may include an utterance acquisition device 200, a mobile terminal 400, and a cognitive ability assessment management server 500.

The utterance acquisition device 200 may include, e.g., an output module 210, an input module 220, a processing module 230, and a communication module 240.

The output module 210 may output a speech according to a plurality of tasks provided to the user 100. As described above, the output module 210 may adjust the magnitude of the speech regarding the output task considering the hearing ability of the user 100 and provide the same.

The input module 220 may obtain a speech according to an answer of the user 100 to a plurality of tasks provided by the output module 210.

The processing module 230 may convert the speech obtained by the input module 220 into utterance data in a digital form. The processing module 230 may adjust the magnitude of at least a portion of the obtained speech and then convert the same into utterance data.

The processing module 230 may remove noise from the speech obtained by the input module 220 through analysis of the frequency band. The processing module 230 may select a portion to be amplified from the speech obtained by the input module 220 through analysis of the frequency band.

The communication module 240 may transmit utterance data generated by the processing module 230 to the mobile terminal 400.

The utterance acquisition device 200 may be implemented in various forms such as a speaker or the like. The utterance acquisition device 200 may be implemented in the form of a wearable device in some cases.

For example, the utterance acquisition device 200 may be implemented as a wearable device including an earphone 201, a microphone 202, a main body 203, and a connection unit 204. The microphone 202 may be attached to the main body 203. The earphone 201 may be connected to the main body 203 through the connection unit 204.

The main body 203 may have a form of surrounding the neck of the user 100. While the main body 203 of the utterance acquisition device 200 is worn on the user 100 while surrounding the neck of the user 100, a task may be provided to the user 100 and the speech by the utterance of the user 100 may be obtained.

The microphone 202 may be implemented to tightly contact the neck of the user 100 to detect vibration caused by the utterance of the user 100 and obtain a speech according to the utterance of the user 100. Even when the utterance ability of the user 100 is degraded, the assessment of cognitive ability through analysis of the utterance data of the user 100 may be performed.

The earphone 201 may be provided in a state of being connected to the main body 203 through the connection unit 204. Use convenience may be provided to the user 100. In some cases, the earphone 201 may be implemented in a form in which a function of measuring the brain waves or electroencephalography (EEG) of the user 100 is added.

Further, the speech acquisition device 200 may provide a hearing aid function that complements hearing so that the user 100 with hearing loss may hear a speech more easily.

Referring to FIG. 7, the configuration of the utterance acquisition device 200 is exemplarily described. The input module 220 may include a plurality of microphones. The input module 220 may use a directional microphone or adopt an algorithm to more efficiently listen to the speech output from an external device such as a smart device. In some cases, an omnidirectional microphone may be used, but when a directional microphone is used, the noise removal effect may be enhanced and the utterer's speech may be captured better.

The processing module 230 may convert the input speech into the frequency domain, process the same, and provide an amplified and noise-removed speech. As an example, the input speech may include environmental noise according to the current location of the user 100, and the environmental noise may include features of various outdoor environments such as an office environment, a subway, a factory environment, etc. As shown in FIG. 7, the feature classification for environmental noise may use information obtained by performing fast Fourier transform on the input signal. Further, the fast Fourier-transformed signal may be amplified with an amplification gain differing according to the frequency domain. Through this amplification, it is possible to transfer a clearer sound to the user 100 (elderly) with poor hearing by making a low sound louder. The amplification method may be similar to the example described with reference to FIG. 4. Further, a function of removing environmental noise may be performed. Accordingly, it is possible to amplify the speech frequency band while removing noise from the received speech. If necessary, the frequency-converted signal may perform spectral enhancement.

The noise removal and speech amplification functions by the processing module 230 may be controlled by, e.g., a remote control device (e.g., a smartphone, a PC, etc.). Remote control may be performed on the compression amplification magnitude for each frequency band, the speech and noise ratio for noise removal, and the echo suppression parameter through the communication module 240.

Further, the utterance acquisition device 200 may provide a speech processing function for increasing the speech recognition rate of the user (100).

Referring to FIG. 8, when the configuration of the utterance acquisition device 200 is exemplarily described, the input module 220 may include at least one microphone. The input module 220 may include a directional microphone or may apply a directional algorithm using a plurality of microphones to efficiently hear the speech by the utterance of the user 100. In some cases, the omnidirectional microphone may be included, but the directional microphone may enhance the noise removal effect and better receive the speech of the utterer. The input module 220 may primarily process the sound obtained through the microphone and then transfer it to the processing module 230. For example, the input module 220 may remove noise from the sound obtained through the microphone or amplify at least a portion of the obtained sound to transfer a clear sound to the processing module 230.

The processing module 230 may remove noise from a speech including noise input from the microphone, or may analyze environmental noise to extract information through classification of an external environment in which the utterer is positioned. The processing module 230 may perform a function of amplifying a small speech of the utterer. Further, the processing module 230 may pass it without performing separate speech signal processing.

The processing module 230 may perform fast Fourier transform on the signal input through the microphone. By analyzing the frequency features of the converted signal, information about the environment in which the utterer is positioned may be provided. For example, as the environment in which the utterer is positioned, a quiet office environment, an environment such as a subway, an external environment, etc. may be analyzed. This information may be used to remove environmental noise other than the speech information about the utterer input through the microphone.

Further, the processing module 230 may increase the recognition rate of the speech recognizer by amplifying the interjections or muttered small speeches input through the microphone in the fast Fourier-transformed signal, and the small speech expressed according to the features of the utterer. When a small speech is amplified, noise included in the speech is amplified together to reduce the speech recognition rate, so it may be preferable to perform noise removal.

The processing module 230 may process and digitize the analog sound received from the input module 220. For example, the processing module 230 may classify the obtained sound and may pass or delay the whole of the sound. The processing module 230 may perform a process of converting the obtained sound into a frequency spectrum and amplifying a portion of the converted sound (DYNAMIC RANGE COMPRESSION (WDRC)) and a process of removing noise (NOISE SUPPRESSION). The processing module 230 may further include a configuration for selectively amplifying the sound in order to further amplify a specific sound. If the processing of the sound is completed, the processing module 230 may convert the sound back into the time domain and output the same. The output sound may be provided as feedback and reflected in the sound output by the processing module 230. The processing module 230 may provide digitized speech data to the communication module 240.

If receiving the digitized speech data, i.e., the utterance data, the communication module 240 may transmit the utterance data to the mobile terminal 400 such as a smart device.

The mobile terminal 400 may include a task-providing module 410, an utterance data collection module 420, and a cognitive ability assessment module 430.

The task providing module 410 may select a task provided to the user 100 through the utterance acquisition device 200 and provide the selected task to the user 100 through the utterance acquisition device 200 according to the test period. The task-providing module 410 may be positioned in the cognitive ability assessment management server 500 in some cases.

The utterance data collection module 420 may obtain utterance data transmitted by the utterance acquisition device 200.

The utterance data collection module 420 may receive utterance data based on a test speech in which noise is removed from the basic speech according to the answer of the user 100 and the magnitude of at least a portion of the basic speech is adjusted.

The utterance data collection module 420 may receive utterance data that has been digitally processed by the utterance acquisition device 200. In some cases, the utterance data collection module 420 may receive an analog speech from the utterance acquisition device 200 and process the received speech into digital utterance data.

The cognitive ability assessment module 430 may produce the assessment result regarding the cognitive ability of the user 100 based on the utterance data.

The cognitive ability assessment module 430 may analyze utterance data obtained by the utterance data collection module 420 and produce the assessment result regarding the cognitive ability of the user 100. The method in which the cognitive ability assessment module 430 produces the assessment result regarding the cognitive ability of the user 100 may be similar to the method performed by the cognitive ability assessment unit 430 included in the cognitive ability assessment device 400 described above.

The cognitive ability assessment module 430 may be positioned in the cognitive ability assessment management server 500 in some cases. That is, the cognitive ability assessment may be performed in the cognitive ability assessment management server 500. In this case, an assessment result may be transferred from the cognitive ability assessment management server 500 to the mobile terminal 400.

As illustrated in FIG. 6, when the task-providing module 410, the utterance data collection module 420, and the cognitive ability assessment module 430 are included in the mobile terminal 400, the cognitive ability assessment management server 500 may provide a function of updating a task that may be provided to the user 100 or a function of storing and managing the assessment result regarding the cognitive ability of the user 100. In some cases, some of the functions performed by the mobile terminal 400 may be performed by the cognitive ability assessment management server 500.

As described above, the mobile terminal 400 and the cognitive ability assessment management server 500 may communicate with each other through the network 600, providing a system capable of easily performing a test on the cognitive ability of the user 100.

Further, in some cases, embodiments of the disclosure may obtain the EEG of the user 100 appearing in the test period, the utterance period, or a period other than the utterance period together with the utterance data of the user 100 to produce the assessment result regarding the cognitive ability of the user 100.

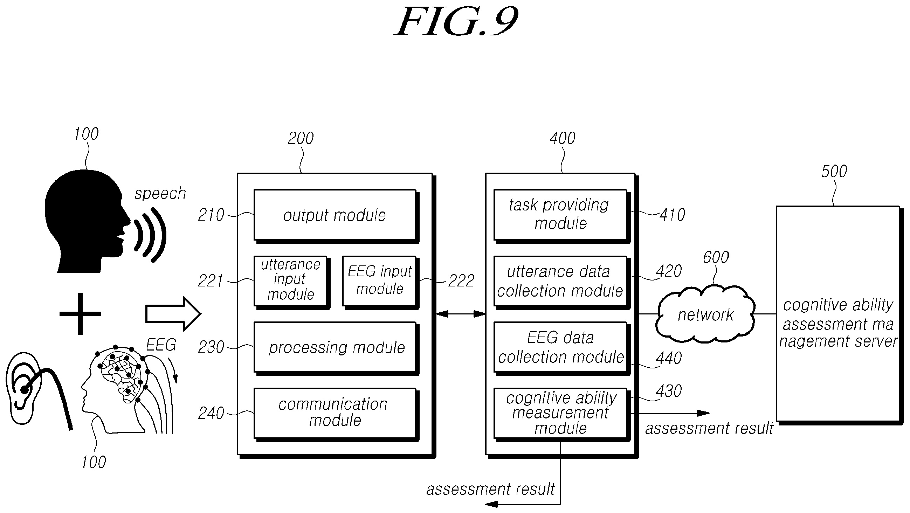

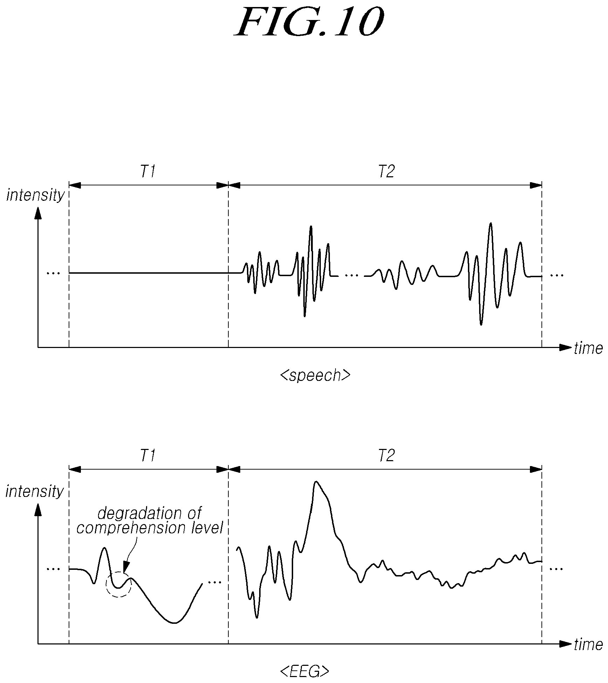

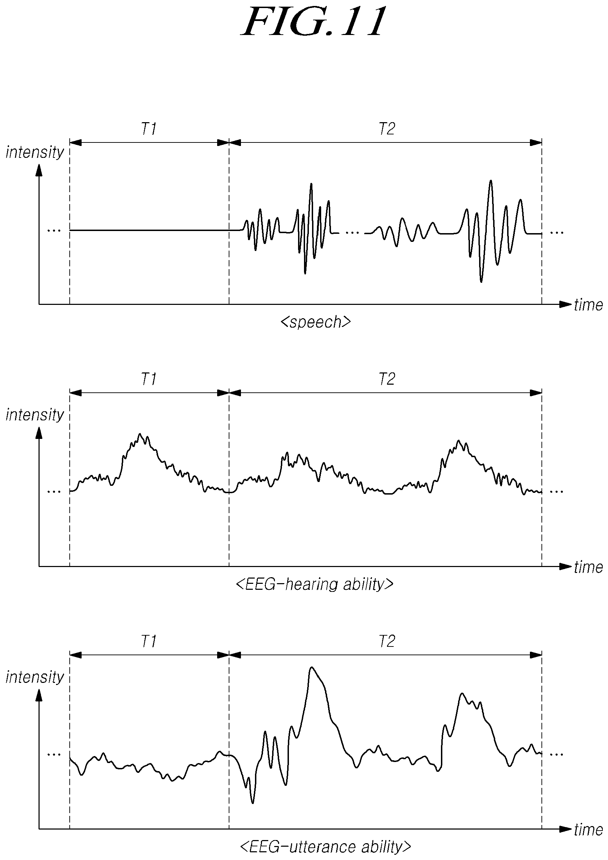

FIG. 9 is a view schematically illustrating still another example of a configuration of a cognitive ability assessment system according to embodiments of the disclosure; FIGS. 10 and 11 are views illustrating examples of a method of performing a cognitive ability assessment by a cognitive ability assessment system according to embodiments of the disclosure; and

Referring to FIG. 9, a cognitive ability assessment system according to embodiments of the disclosure may include an utterance acquisition device 200, a mobile terminal 400, and a cognitive ability assessment management server 500.

Similar to the example described with reference to FIG. 6, the utterance acquisition device 200 may include an output module 210, a processing module 230, and a communication module 240. The utterance acquisition device 200 may include an utterance input module 221 and an EEG input module 222. The utterance acquisition device 200 may obtain a speech by the utterance of the user 100 and obtain an EEG of the user 100. The utterance acquisition device 200 may obtain the EEG of the user 100 through a portion connected to the head or ear of the user 100.

The utterance acquisition device 200 may obtain the EEG of the user 100 during at least a partial period of the period in which the speech by the utterance of the user 100 is obtained. Alternatively, the utterance acquisition device 200 may obtain the EEG of the user 100 during a period distinguished from the period during which the speech by the utterance of the user 100 is obtained.

The utterance input module 221 and the EEG input module 222 may obtain the speech and EEG of the user 100. The communication module 240 may transmit the utterance data according to the speech of the user 100 and the EEG data according to the EEG to the mobile terminal 400.

The mobile terminal 400 may include a task providing module 410, an utterance data collection module 420, and a cognitive ability assessment module 430, and may further include an EEG data collection module 440.

The mobile terminal 400 may use the obtained EEG data in various ways while producing the assessment result regarding the cognitive ability of the user 100 based on the utterance data.

For example, referring to FIG. 10, a task may be provided during a test period including a first test period T1 and a second test period T2, and a test for assessing the cognitive ability of the user 100 may be performed.

The main test for assessing the cognitive ability of the user 100 may be performed during the second test period T2. Guidance for the main test may be provided during the first test period T1.

First EEG data may be obtained during the first test period T1. Second EEG data may be obtained during the second test period T2.

The first EEG data may be data obtained from the EEG related to the comprehension level of the user 100. The task providing module 410 or the cognitive ability measurement module 430 may identify the comprehension level of the user 100 of the guidance for the main test based on the first EEG data. Based on the first EEG data, whether to perform the main test during the second test period T2 or the difficulty level, type, or the like of the main test may be determined.

The second EEG data may be data obtained from the EEG related to the utterance ability of the user 100. The cognitive ability measurement module 430 may produce the assessment result regarding the cognitive ability of the user 100 using the analysis result regarding the second EEG data together with the analysis result regarding the utterance data.

As such, when the utterance acquisition device 200 is implemented in a form capable of measuring the EEG of the user 100 at the same time, a test method may be determined using the EEG data collected during the first test period T1 and the second test period T2, or the assessment result regarding the cognitive ability may be produced.

Further, when the test for the cognitive ability assessment is performed during the first test period T1, the EEG data obtained during the first test period T1 may also be used to produce the assessment result regarding the cognitive ability of the user 100. In this case, similar to the utterance data, different weights may be applied to the EEG data obtained during the first test period T1 and the second test period T2 and used to produce the assessment result regarding the cognitive ability.

Alternatively, another ability of the user 100 may be measured through the EEG data and used to produce the assessment result regarding the cognitive ability of the user 100. Like as described above, the cognitive ability assessment may be performed in the mobile terminal 400, and also, the cognitive ability assessment may be performed in the cognitive ability assessment management server 500. In a case that the cognitive ability assessment is performed in the cognitive ability assessment management server 500, an assessment result may be transferred from the cognitive ability assessment management server 500 to the mobile terminal 400.

For example, referring to FIG. 11, the utterance acquisition device 200 may obtain the speech by the utterance of the user 100 and simultaneously obtain the EEG of the user 100.

The utterance acquisition device 200 may simultaneously obtain a plurality of types of EEGs.

For example, the utterance acquisition device 200 may obtain the EEG related to the hearing ability of the user 100 during at least a partial period of the first test period T1 and the second test period T2.

Further, the utterance acquisition device 200 may obtain the EEG regarding the utterance ability of the user 100 during at least a partial period of the first test period T1 and the second test period T2.

The mobile terminal 400 may produce the assessment result regarding the cognitive ability of the user 100 using the EEG data regarding the hearing ability obtained during the first test period T1 and the second test period T2. The mobile terminal 400 may produce the assessment result regarding the cognitive ability of the user 100 using the EEG data regarding the utterance ability obtained during the first test period T1 and the second test period T2.

When the hearing activity of the user 100 is mainly performed during the first test period T1 and the utterance activity of the user 100 is mainly performed during the second test period T2, the assessment result regarding the cognitive ability of the user 100 may be produced using the EEG data regarding the hearing ability of the user 100 obtained during the first test period T1 and the EEG data regarding the utterance ability of the user 100 obtained during the second test period T2.

Alternatively, the assessment result regarding the cognitive ability of the user 100 may be produced using both the EEG data regarding the hearing ability and the EEG data regarding the utterance ability obtained during the first test period T1 and the second test period T2. In this case, in some cases, a different weight may be applied to each EEG data for each test period and used to produce the assessment result.

The mobile terminal 400 may produce the assessment result regarding the cognitive ability of the user 100 using the utterance data obtained from the user 100 and the EEG data together.

When the cognitive ability is degraded, the utterance ability may be degraded or the hearing ability may be degraded. Or, the speech ability and the hearing ability may be degraded simultaneously. Accordingly, it is possible to measure the degradation of the cognitive ability of the user 100 through the test of the speech ability and the hearing ability.

Further, in some cases, the degree of degradation of the speech ability and the degree of degradation of the hearing ability may be different. For example, for growing persons, the rate of development of hearing ability may be faster than the rate of development of utterance ability. On the other hand, for the elderly, the rate of degeneration of utterance ability may be faster than that of hearing ability. Alternatively, unlike the developmental process, the rate of degeneration of the two abilities may be similar. Alternatively, the degeneration rate of hearing ability may be fast during degeneration.

In this way, it is possible to determine the state of cognitive ability through whether or not hearing ability is degraded. In some cases, the state of the cognitive ability may be divided into several levels based on the difference between the hearing ability and the utterance ability, and the assessment result regarding the cognitive ability of the user 100 may be produced. Further, when considering whether the hearing ability is degraded when determining the state of the cognitive ability, data according to the level of the hearing aid function provided by the speech acquisition device 200 may be used. The assessment result regarding the cognitive ability may be produced by reflecting the data regarding the degree of degradation of the hearing ability according to the level of the hearing aid function.

Since the EEG data is used together with the utterance data, the accuracy of the cognitive ability assessment by the cognitive ability assessment system according to embodiments of the disclosure may be enhanced.

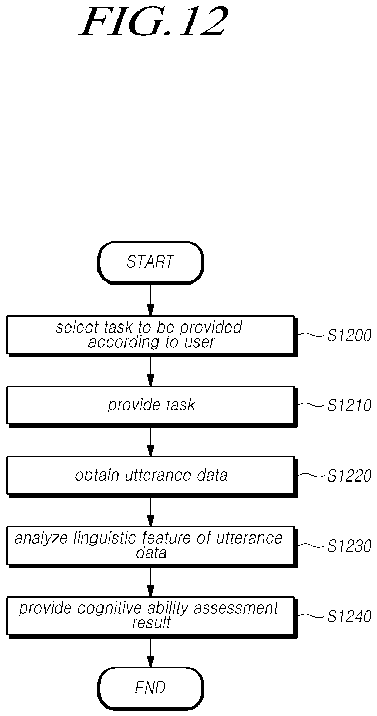

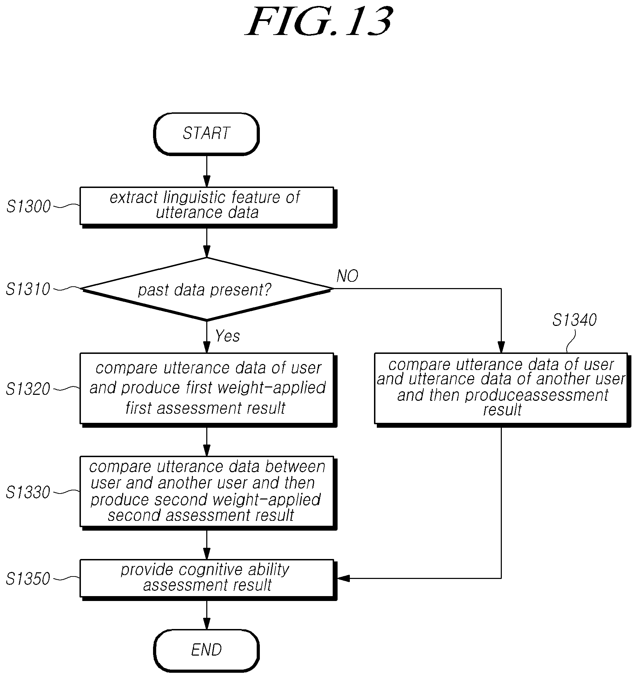

FIGS. 12 and 13 are flowcharts illustrating examples of a process of a cognitive ability assessment method according to embodiments of the disclosure.

Referring to FIG. 12, the cognitive ability assessment system may select a task to be provided to the user 100 according to the user 100 (S1200). The difficulty level and type of the provided task, the content of the question included in the task, and the like may be selected according to the user 100.

Tasks may be provided by the cognitive ability assessment system (S1210).

A answer of the user 100 to the task may be generated, and accordingly, utterance data of the user 100 may be obtained (S1220).

The cognitive ability assessment system may analyze the linguistic features of the utterance data (S1230). The cognitive ability assessment system may provide an assessment result of the cognitive ability of the user 100 based on the analysis of the linguistic features of the utterance data (S1240).

Alternatively, in some cases, the cognitive ability assessment system may provide the assessment result regarding the cognitive ability of the user 100 based on the linguistic and acoustic features of the utterance data.

The linguistic features of the utterance data may refer to features such as sentences and words by the utterance of the user 100. The acoustic features of the utterance data may refer to features related to the intensity, frequency, tone, etc. of the speech by the utterance of the user 100.

The assessment result regarding the cognitive ability of the user 100 may be produced using the linguistic and acoustic features of utterances appearing in normal people, MCI patients, patients with dementia, etc. Further, based on the acoustic features of the utterance data, diseases different from MCI such as depression may be distinguished, and the assessment result regarding the cognitive ability of the user 100 may be produced.

The linguistic features extracted through the utterance data of the user 100 may be compared with various pre-stored reference data, and the assessment result regarding the cognitive ability of the user 100 may be produced.

Referring to FIG. 13, the cognitive ability assessment system may extract linguistic features of the utterance data (S1300). In some cases, as described above, acoustic features of the utterance data may also be used.

The cognitive ability assessment system may identify whether there is past data of the user 100 being tested (S1310).

When the past data of the user 100 is present, the cognitive ability assessment system may produce the first assessment result by comparing the utterance data of the user 100 (S1320). Through utterance data analysis, a change in the utterance ability of the user 100 may be identified, and the first assessment result may be produced based on the change. The cognitive ability assessment system may apply the first weight to the first assessment result.

The cognitive ability assessment system may assess the second assessment result after comparing features of the utterance data of the user 100 with them of the utterance data of another user (S 1330). The utterance data of the other user may be utterance data of another user who has been tested, or utterance data collected from a user with a disease such as MCI. The cognitive ability assessment system may apply the second weight to the second assessment result.

When the past data of the user 100 is not present, the cognitive ability assessment system may produce the assessment result after comparing the utterance data between the user 100 and the other user (S 1340).

The cognitive ability assessment system may provide the assessment result regarding the cognitive ability based on the comparison result between features of the utterance data of the person and them of the utterance data comparison result between the person and another person (S1350).

The first weight applied to the first assessment result according to the comparison between features of the utterance data of the person and the second weight applied to the second assessment result according to the comparison between the utterance data of the person and the other person may be set variously.

For example, based on the age of the user 100, the degree of change in the cognitive ability, the degree of risk, or the like, the change rate of cognitive ability of the user 100 may be prioritized, or the difference between the user and another person may be prioritized, thereby making it possible to more finely determine whether the user 100 has a risk of MCI.

According to embodiments of the disclosure, it is possible to easily measure the cognitive ability of the user 100 using the utterance data obtained by performing tasks in daily life or public places when a wearable device having noise removal and selective amplification functions is used. In other words, according to embodiments of the disclosure, the accessibility of the user 100 is excellent, and a test may be conveniently performed anytime, anywhere using the wearable device.

According to embodiments of the disclosure, it is possible to increase the speech recognition effect by applying a noise removal technique.

According to embodiments of the disclosure, the utterer's detailed information may be collected through a speech amplification function, increasing analysis capability.

According to embodiments of the disclosure, it is possible to select an optimal test question set considering, e.g., the subject or utterer's individual intelligent ability, language cognitive ability, or the like by selecting selective questions for each task.

According to embodiments of the disclosure, it is possible to select a question set by providing a physical sensor, time-based question start and end information.

According to embodiments of the disclosure, as the test time may be reduced through a selective question test scheme according to individual circumstances, the test may be performed with the significantly reduced fatigue of the elderly.

According to embodiments of the disclosure, it is possible to quantify utterance information about each question. Several questions may be asked for each task, and the utterance for each question may be quantified, and be presented as an average value. The information for each task may be combined.

The above-described embodiments are merely examples, and it will be appreciated by one of ordinary skill in the art various changes may be made thereto without departing from the scope of the present invention. Accordingly, the embodiments set forth herein are provided for illustrative purposes, but not to limit the scope of the present invention, and should be appreciated that the scope of the present invention is not limited by the embodiments. The scope of the disclosure should be construed by the following claims, and all technical spirits within equivalents thereof should be interpreted to belong to the scope of the disclosure.

### CROSS-REFERENCE TO RELATED APPLICATION

The instant patent application claims priority under 35 U.S.C. 119(a) to Korean Patent Application No. 10-2022-0105435, filed on August 23, 2022, in the Korean Intellectual Property Office, the disclosure of which is herein incorporated by reference in its entirety. The present patent application claims priority to other applications to be filed in other countries, the disclosures of which are also incorporated by reference herein in their entireties.

## Claims

1. A cognitive ability assessment device, comprising:
a task provider providing a user with a plurality of tasks during a first test period and a second test period after the first test period;
an utterance data collector obtaining utterance data in a digital form, converted from a test speech in which a magnitude of at least a portion of a basic speech according to a user's answer to at least one of the plurality of tasks is adjusted; and
a cognitive ability measurer producing an assessment result regarding the user's cognitive ability based on at least one linguistic characteristic of the utterance data.

2. The cognitive ability assessment device of claim 1, wherein the user's ambient noise is collected during the first test period, and a noise frequency band is extracted through frequency band analysis of the ambient noise.

3. The cognitive ability assessment device of claim 2, wherein the basic speech is collected during the second test period and, after noise is removed from the basic speech based on the noise frequency band, the magnitude of the at least a portion of the basic speech is adjusted.

4. The cognitive ability assessment device of claim 1, wherein a first portion of the basic speech is amplified, and a second portion of the basic speech is not amplified.

5. The cognitive ability assessment device of claim 1, wherein the magnitude of the at least a portion of the basic speech is adjusted in an external device.

6. The cognitive ability assessment device of claim 1, wherein first utterance data based on the basic speech is obtained during the first test period, and second utterance data based on the basic speech is obtained during the second test period, and wherein a first weight is applied to the first utterance data, and a second weight is applied to the second utterance data and are used to produce the assessment result regarding the user's cognitive ability.

7. The cognitive ability assessment device of claim 1, wherein the plurality of tasks are provided to the user in a speech form, and wherein a magnitude of the speech regarding the plurality of tasks is adjusted according to the user's hearing ability.

8. The cognitive ability assessment device of claim 1, wherein the cognitive ability measurer produces the assessment result regarding the user's cognitive ability based on the at least one linguistic characteristic of the utterance data and the user's hearing ability.

9. The cognitive ability assessment device of claim 1, wherein the cognitive ability measurer produces the assessment result regarding the user's cognitive ability based on a first assessment result produced by comparing the utterance data with the user's past utterance data and a second assessment result produced by comparing the utterance data with average utterance data of other users than the user.

10. The cognitive ability assessment device of claim 1, wherein the cognitive ability measurer produces the assessment result regarding the user's cognitive ability based on at least one of a number of words used by the user during a preset period included in the utterance data, a type of the words used by the user during the preset period, a number of duplicate words used by the user during the preset period, a number of words used by the user for each section of the preset period, an utterance period and a pause period included in the preset period, a variation rate of a length of at least one of the utterance period or the pause period, or a variation in a proportion of the pause period.

11. The cognitive ability assessment device of claim 1, wherein the task provider provides a task leading to an answer in which some content of information recognized by the user is changed.

12. The cognitive ability assessment device of claim 1, wherein the task provider provides a task generated based on at least one of individual information or common information recognized by the user.

13. The cognitive ability assessment device of claim 1, wherein the utterance data based on the user's answer to the at least one task among the plurality of tasks is not used to produce the assessment result regarding the user's cognitive ability.

14. The cognitive ability assessment device of claim 1, wherein the task provider adjusts a task provided after the user's answer based on the user's answer.

15. A mobile terminal, comprising:
a task providing module providing a user with a plurality of tasks during a test period; and
an utterance data collection module obtaining utterance data in a digital form, converted from a test speech in which a magnitude of at least a portion of a basic speech according to a user's answer to at least one of the plurality of tasks is adjusted.

16. The mobile terminal of claim 15, further comprising a cognitive ability measurement module producing an assessment result regarding the user's cognitive ability based on at least one linguistic characteristic of the utterance data.

17. An utterance acquisition device, comprising:
an output module outputting a speech according to a plurality of tasks provided to a user, a magnitude of the speech being adjusted according to the user's hearing ability;
an input module obtaining a basic speech according to the user's answer to at least one of the plurality of tasks; and
a processing module processing utterance data in a digital form based on the basic speech.

18. The utterance acquisition device of claim 17, wherein the processing module analyzes a frequency band of the basic speech, adjusts a magnitude of at least a portion of the basic speech through the analysis of the frequency band to generate a test speech, and converts the test speech into utterance data in the digital form.

19. The utterance acquisition device of claim 17, wherein the processing module collects the user's ambient noise during a first period and analyzes a frequency band of the ambient noise to extract a noise frequency band.

20. The utterance acquisition device of claim 19, wherein the processing module collects the basic speech during a second period after the first period, removes noise based on the noise frequency band from the basic speech, and then adjusts the magnitude of the at least a portion of the basic speech.
